# EUROPEAN PATENT SPECIFICATION

(11) **EP 3 380 081 B1**
(45) Date of publication and mention of the grant of the patent: **16.06.2021**
(21) Application number: 15816858.3
(22) Date of filing: 23.11.2015
(51) Int. Cl.: A61K 9/127, A61K 8/04, A61Q 19/00

(54) **METHOD FOR PREPARING A STABLE CONTROLLED-RELEASE PROPOLIS COLLOIDAL DISPERSION SYSTEM FOR VARIOUS USES**
VERFAHREN ZUR HERSTELLUNG EINES STABILEN KOLLOIDALEN PROPOLIS-DISPERSIONSSYSTEMS MIT KONTROLLIERTER FREISETZUNG FÜR VERSCHIEDENE VERWENDUNGEN
PROCÉDÉ DE PRÉPARATION D'UN SYSTÈME STABLE DE DISPERSION COLLOÏDALE DE PROPOLIS À LIBÉRATION CONTRÔLÉE, POUR DES UTILISATIONS VARIÉES

(43) Date of publication of application: 03.10.2018
(73) Proprietor: Apivita S.A., 19003 Markopoulo (GR)
(72) Inventor: GARDIKIS, Konstantinos, 112 53 Athens (GR); KOUTSIANAS, Nikolaos, 194 00 Koropi (GR); PATERA, Anna, 115 25 Athens (GR); DRAGANI, Panagiota, 111 45 Athens (GR); TSOUKALAS, Anagnostis- Ioannis, 115 27 Athens (GR); LETSIOU, Sofia, 162 33 Byronas (GR)
(74) Representative: Prokopiou, Dimitrios
(86) International application number: PCT/GR2015/000059
(87) International publication number: WO 2017/089842

(56) References cited:
- CN-A- 102 138 941
- CN-B- 103 191 157
- JP-B2- 2 945 852

## Description

### Technical field

The present invention relates to a method of producing a stable controlled-release propolis colloidal dispersion system and its use as an antioxidant, angiogenic, photoprotective, antimicrobial and immunostimulatory agent, alone or incorporated in cosmetic or pharmaceutical formulations, which is characterized by the fact that the components of propolis are extracted and at the same time encapsulated in combinatorial liposome-cyclodextrin carriers so that a controlled release rate of the components is obtained.

### Technological background

Until now, such a product or a method for the production thereof has not been known. In particular, propolis is a resinous substance which is produced by bees and presents many challenges in respect to its extraction and formulation. It exhibits antimicrobial, immunostimulatory and antioxidant activity and is employed in the preparation of functional foods and cosmetics as well as in traditional medicine. Its composition varies depending on the flora of the foraging region of the bees and the collection season. It contains ca. 50% resins, 30% waxes, 10% aromatic components, 5% pollen and 5% various other components. The bioactive components of propolis are polyphenols, terpenes, steroids, as well as sugars and aminoacids. Major polyphenols are flavonoids and phenolic acids. The complexity of the structure of propolis combined with the variable composition depending on the region and the collection season make both propolis and its extracts particularly difficult to formulate.

The extraction of propolis usually has a low yield in active component concentration. In order to increase the extraction yield, various extraction methods have been developed as well as methods of encapsulating its components in various carriers.

As regards extraction methods, either soaking in an extraction solvent for large time periods is performed, or higher extraction temperatures or organic solvents are used. These methods have the serious disadvantage that high temperatures, prolonged time and the use of high-energy techniques, such as microwave- or ultrasound-assisted extraction may lead to decomposition of the labile components of propolis, mainly of the polyphenols, and deterioration of the quality of the final extract.

Common solvents used for the extraction include various alcohols such as methanol and ethanol. Methanol presents the problem that -despite being an effective solvent for the active components of propolis- it is extremely toxic, therefore it is only used for research purposes. Ethanol is also an effective solvent for the active components of propolis, however it is involved in the rupture of the skin and local irritations, thus it is not indicated for topical administration.

Apart from the solubility of the components, the extraction of propolis presents other problems which have been addressed by the use of carriers for bioactive substances.

A limiting factor for the topical use of propolis extracts is the bioavailability of its components and the penetration depth in the skin. Polyphenols, which are responsible for the antioxidant and immunostimulatory action of propolis, depending on their physicochemical properties and in particular the lipophilicity thereof, accumulate upon topical delivery mainly on the corneal layer (Alonso et al., 2014, Abla et al., 2013), while a minor portion penetrates into the deeper layers of the skin.

Another serious problem is the protection of polyphenols against physical, chemical and biological decomposition due to environmental factors. As it is known in the literature, polyphenols oxidize easily and may leave black spots upon deposition on the skin, become malodorous -which limits their topical use- and lose their activity due to the decomposition (Munin and Edwards-Levy, 2011).

Finally, another problem is the duration of the contact of the polyphenols with the skin cells or mucosa, especially in cases when prolonged action against microorganisms and/or anti-oxidant protection and/or photoprotective and/or angiogenic action are required. As regards prolonged antimicrobial action, controlled-release systems of propolis (De Luca et al.,2014, Franca et al.,2014, Balata et al.,2014) have been described which either use ethanol as solvent -with the above-mentioned toxicity problems- or synthetic polymers. As regards controlled release of anti-oxidants, a system has been described which however relates to a final formulation and not a starting material which may be used alone or as a component of multiple final formulations for use on the skin (Zilius et al.,2013) as well as a nano-emulsion with photoprotective action on the skin which however contains -in addition to propolis- lycopene (Butnariu et al.,2011).

Also, until now, for the increase in solubility, bioavailability and protection of the components of propolis, carriers for bioactive substances such as cyclodextrins or liposomes have been used.

It is known from the literature that the formation of inclusion complexes of bioactive substances in cyclodextrins provides advantages, such as an increase in water solubility, protection against oxidation and protection against decomposition of the molecules, and therefore in the past complexes of polyphenols of propolis with cyclodextrins have been formed.

Encapsulation of polyphenols in liposomes has also been used previously (Tao et al., 2014, Yuan et al.,2013). In these cases, propolis was subjected to ethanol extraction, with all the above-mentioned problems, and then liposomal systems encapsulating polyphenols were formed. This is a multi-step procedure.

The present invention aims at eliminating the above disadvantages. In particular, the present invention allows for the first time simultaneous extraction and encapsulation of components of propolis in a combinatorial liposome-cyclodextrin system, which consists exclusively of natural and non-toxic ingredients and which presents controlled release of the encapsulated polyphenols of propolis.

More specifically the present invention exploits the particular properties of both carriers: the ability of the cyclodextrins to enclose polyphenols and enhance their permeability through the skin (Cutignelli et al.,2014) as well as the ability of the liposomes to encapsulate large quantities of components of various degrees of polarities, for topical transport of components and control of their release rate. At the same time, the extraction of the polyphenols and their encapsulation in the combinatorial system takes place in a single vessel.

Aim of the present invention is the preparation of a stable propolis colloidal dispersion system with a novel method where the components of propolis are extracted and at the same time encapsulated in combinatorial liposome-cyclodextrin carriers. The system, under suitable conditions, allows controlled release of the components while under suitable storage conditions it keeps the components encapsulated in the combinatorial system. The final preparation is suitable for antioxidant, photoprotective, antimicrobial, angiogenic and immunostimulatory use, either alone or after incorporation in cosmetic or pharmaceutical formulations.

The advantages and novel features of the method are the following:
- It is a particularly simple method for producing a complex combinatorial propolis delivery system. The procedure of forming inclusion complexes between hydroxypropyl-b-cyclodextrin and the active components of propolis, as well as of encapsulating propolis in liposomes, either in free form or in the form of a complex with cyclodextrin, is performed during extraction in a single vessel.
- The time required for the formation of the colloid is small and energy supply is minimal, thus the final production cost is kept proportionally to very low levels.
- The simultaneous extraction by means of the specific different carriers (liposomes and cyclodextrins) ensures a very high yield in extraction of polyphenols. The encapsulation efficiency of the system in polyphenols is higher than 95%.
- The interaction of the two carriers with the polyphenols of propolis ensures controlled and sustained release of the components of propolis under suitable conditions. Under storage conditions the components are kept protected in their interior.
- The combination of the components and the lipid composition of the liposomes are such that fusion and aggregation of liposomes are avoided, resulting to excellent stability of the system both physicochemically (mean hydrodynamic diameter, z-potential, polydispersity index, pH) and in regards to the retention of the polyphenols in the liposomes during storage.
- Only components that are safe and compatible to the skin and mucosa are used.
- The final extract is titrated to total polyphenols.
- The final extract has a specific effect on the metabolism of human fibroblasts.
- The colloidal system is self-sustaining and does not require the addition of preservatives, despite its large water content. This is due to the extremely high content in polyphenols, which are responsible for the antimicrobial capacity of propolis.
   - As a result of the double encapsulation in liposomes and cyclodextrins, the components of the colloidal system are protected against oxidation or decomposition induced by light or temperature increase. Also, for the same reason, they are protected against interaction with other components, when the colloidal system is used as a component of cosmetic or pharmaceutical formulations.
- The colloidal system may be used directly on the human skin and mucosa, or encapsulated in cosmetic or pharmaceutical formulations for topical or systematic delivery. In cosmetic or pharmaceutical formulations it may provide clear liquids as required, e.g. in the preparation of gels.
- The colloidal system may be used in a suitable formulation as food supplement, nutraceutical and as a component in functional foods.
- The colloidal system of propolis is considered effective for antioxidant, angiogenic, photoprotective, antimicrobial and immunostimulatory use.

### Disclosure of the invention

In order to make the present invention fully understood by those skilled in the art, a detailed description of the preparation method of the colloidal dispersion system of propolis in combinatorial liposome/cyclodextrin carriers is given.

In order to prepare a system, propolis is initially micronized (<1 mm) after being frozen for 24 hours. Any propolis may be used without previous treatment, as long as it contains total polyphenols >1600 mg/l gallic acid after dissolution of 10 %(w/w) propolis in ethanol and subsequent measurement in a spectrophotometer by the Folin-Ciocalteau method. Thereafter, the micronized propolis is dispersed at a rate of 1 kg/min in the solvent system under stirring at 500-3000 rpm. The propolis concentration is in the range of 7.5 to 28 %(w/w) depending on polyphenol content. The system of extraction solvents consists of deionized water and either 1,3-propanediol or glycerol at a ratio of 1,3 propanediol (or glycerol)/water: 15/85 to 80/20. In the deionized water, hydroxypropyl-β-cyclodextrin or β-cyclodextrin has been predissolved at 2-10% w/w.

The quality specification of the deionized water is <=1 µS/cm at 25°C, which meets the specification of European Pharmacopoeia for the preparation of parenteric pharmaceutical products. This quality is necessary for full absence of loads in the final formulation which would otherwise cause the lipid membranes of the liposomes to aggregate and finally lead to a decrease in the product stability.

The preparation of the deionized water used in our invention is as follows:

Tap water is introduced in the raw-water tank (volume 2 m³) by a suitable pumping system, passed through an automatic turbidity filter to remove turbidity and solid particles and activated carbon to remove chlorine and organic load and then an antiscalant is dosed to remove its hardness. Before its input in the central plant of the reverse-osmosis unit, it is passed through a 1-micron cartridge filter.

The fully treated water for use in reverse osmosis is introduced in the reverse-osmosis unit of a productivity of 350 It/h with a recovery of 70%. The water produced from the unit is kept in a stainless-steel 5-m³ tank. From this tank, water is supplied by a suitable pumping system to the deionizer and is directly supplied to the tank of extraction through U.V. radiation. In order to avoid stagnant water in the network, water is circulated continuously with return to the tank.

As the system of propolis/cyclodextrin/solvents is under intense stirring (2000-3000 rpm) for 30-45 min and at a temperature of 20°C to 38°C, a liposomal suspension is added thereto at a proportion of 0.3 to 3.5% (w/w). If required, pH adjustment in the range of pH 5-8 is previously performed, depending on the propolis used. The liposomal suspension added consists of large unilamellar liposomes, lipidic bilayers and natural 1,3-propanediol. The liposomal suspension is at a temperature higher than the phase transition temperature of its phospholipids and its lipidic composition is:
- 50%-90% phosphatidylcholine
- 2%-10% phosphatidylethanolamine
- 1%-3% lysophosphatidylcholine
- 1%-3% phosphatidylinositol
- 1%-3% phosphatidic acid
- 0%-40% cholesterol
- 0-20% cholate salts

Stirring is performed for 1 to 4 hours, and at the same time the following parameters are measured:
- Total polyphenol content by the Folin-Ciocalteau method
- % Encapsulation efficiency

When the above-mentioned parameters reach the desired values, the colloidal system is filtrated through suitable filters (cartridge filters) having a pore size of 0.45 µm and pH is adjusted to 5,0-8.0, as required, and stirring is ended.

The colloidal system is acceptable if its application on NHDF (primary human skin fibroblasts) leads to an increase in their vitality by at least 100% vs. control. The increase in the vitality of cells is represented by the increase in ATP (adenosine triphosphate) therein.

Thereafter, the size of dispersed particles and the release rate of the polyphenols of propolis is measured in a buffer solution at pH 7.2 at 37°C and the colloid is stored in a dark-coloured container at a temperature of 5-7°C, where it is kept stable for 2 years.

The size of the liposomes produced by this method is in the range of 70 to 700 nm and the polydispersity index is less than 0.5 (<0.5), which is a requirement for their stability. The cumulative release of the polyphenols at pH 7.2 and at a temperature of 37°C is 25-60% for 8 hours while the system releases practically all the encapsulated polyphenols within 24 hours.

In order to make the present invention fully understood, the following examples are presented:

### Example 1

Propolis which was micronized (<1 mm) after deep freezing for 24 hours is dispersed in the solvent system consisting of water and natural 1,3-propanediol at a ratio 1,3-propanediol/water: 45/55. In the aqueous phase hydroxypropyl-β-cyclodextrin has been predissolved to a content of 6,45%.

As the system is under intense stirring (3000 rpm) and at a temperature of 20°C, a liposomal suspension is added thereto at a proportion of 3.5% (w/w). The end point of the extraction is determined by the values of Total polyphenols and Encapsulation efficiency % determined, and is confirmed by the value of ATP increase vs control in NHDF % as shown in the Table 1.

**Table 1**

| Parameter | Value |
|---|---|
| Total polyphenols* | >1600 ± 80 (mg/l gallic acid) |
| Encapsulation efficiency % | >95 |
| ATP increase vs control in NHDF % | >100 |

| | |
|---|---|
| • The starting material of propolis should contain total polyphenols >1600 mg/l gallic acid after dissolution of 10 %(w/w) propolis in ethanol. | |

The final colloidal suspension after filtration presents the values given in table 2

**Table 2**

| Parameter | Value |
|---|---|
| Mean hydrodynamic particle diameter | 100-250 nm |
| Polydispersity index | <0.5 |
| Cumulative release in 8 hours % | 25-60 |

### Example 2

Propolis which was micronized (<1 mm) after deep freezing for 24 hours is dispersed in the solvent system consisting of water and natural 1,3-propanediol at a ratio 1,3-propanediol/water: 25/75. In the aqueous phase hydroxypropyl-β-cyclodextrin has been predissolved to a content of 4,45%.

As the system is under intense stirring (3000 rpm) and at a temperature of 35°C, a liposomal suspension is added thereto at a proportion of 1.5% (w/w). The end point of the extraction is determined by the values of Total polyphenols and Encapsulation efficiency % determined, and is confirmed by the value of ATP increase vs control in NHDF % as shown in the Table 3.

After completion of the extraction, propolis is filtrated to remove insoluble components.

**Table 3**

| Parameter | Value |
|---|---|
| Total polyphenols* | >1600 ± 80 (mg/l gallic acid) |
| Encapsulation efficiency % | >95 |
| ATP increase vs control in NHDF % | >100 |

The starting material of propolis should contain total polyphenols >1600 mg/l gallic acid after dissolution of 10 %w/w propolis in ethanol.

The final colloidal suspension after filtration presents the values given in table 4

**Table 4**

| Parameter | Value |
|---|---|
| Mean hydrodynamic particle diameter | 100-350 nm |
| Polydispersity index | <0.5 |
| Cumulative release in 8 hours % | 25-60 |

The determination of the mean hydrodynamic diameter and Polydispersity index is effected by Dynamic Light Scattering.

An in vitro study of the release of the polyphenols of propolis was performed by means of dialysis sacks:
A specific quantity of the colloid is placed in the dialysis sacks of a MWCO=1000. The sack is placed in distilled water having pH=7.2 and a temperature of 37°C under mild stirring. On specific time points, samples are taken and their polyphenol concentration is measured, while the water quantity removed is replaced by distilled water having pH=7.2 and a temperature of 37°C to maintain the tank conditions.

The prepared colloidal system of propolis dispersion in combinatorial liposome/cyclodextrin carriers presents advantageous properties in various uses, including for example antioxidant, angiogenic, photoprotective, antimicrobial and immunostimulatory properties.

In particular, it was shown that the prepared colloidal system of propolis did not show cytotoxicity on skin fibroblasts at any concentration. On the contrary, 100% increase in the ATP levels of fibroblasts with the colloidal system of propolis (0.01%, 0.1%, 1%) compared to the ATP levels of the untreated fibroblasts (p<0.05)(barchart 1). Also the colloidal system of propolis of the present invention at a concentration of 1%, protects the cells against photooxidative stress. In particular, after the relevant experimental procedure, an increase in the vitality of the cells (ATP increase) with the colloidal system of 1% propolis under photooxidative stress conditions (UVA radiation) in relation to untreated cells was observed. This is linked to the antioxidant capacity of the colloidal system of 1% propolis as well as to its protective role against premature aging of the cells (photo-aging).

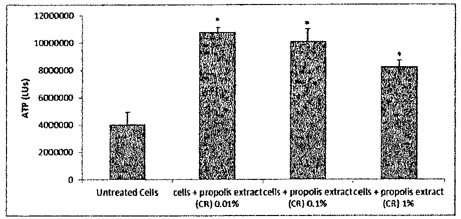

**Barchart 1**. ATP levels in untreated cells, cells with added colloidal system of propolis (0.01%, 0.1%, 1%) (cells+propolis CR 0.01, 0.1 and 1%) and untreated cells. *Statistically significant difference, Student's t-test (p<0.05).

In particular, ultraviolet radiation is a major cause of oxidative stress for the skin. Apart from the generation of free radicals, ultraviolet radiation affects the defense enzymes of the skin against oxidation, making the skin more vulnerable to permanent cellular damage (acceleration of skin aging). When the skin is exposed to ultraviolet light for a long time without protection with sunscreen filters, skin protection depends exclusively on the endogeneous antioxidant defense systems. In our experimental procedure, photooxidative stress was simulated. Therefore, a UV lamp with a wavelength of 365 nm and a radiation dose of 5 J/cm² was used. The UV lamp was used as means to form free radicals. The said experimental procedure includes the following steps:
- Incubation of the colloidal system of 1% propolis on the NHDF cells for 48 hours
- Removal of cell medium and washing with phosphate/salt (PBS) buffer solution to avoid intramolecular reactions
- Addition of phosphate/salt (PBS) buffer solution and activation of the UV lamp at a wavelength of 365 nm for 20 minutes,
- Addition of cell medium
- Measurement of the viability of the cells based on ATP.

With the specific experimental procedure, the previous results were confirmed.
- The colloidal system of 1% propolis causes advantageous on the transcriptomic profile of skin fibroblasts, so that it has an antimicrobial and immunostimulatory action and influences angiogenesis.
- Last but not least, we evaluated for potential skin irritancy of colloidal system of propolis (1%) as well as cosmetic formulation containing of this extract. For this reason we use the reconstructed human epidermal model EpiDerm. This model has been manufactured by MatTek Corporation (Ashland, MA,USA) since 1993. The model consists of human-derived epidermal keratinocytes, which have been cultured to form a multi-layered, highly differentiated model of human epidermis. The tissue exhibits organized basal, spinous, granular layers, and multi-layered stratum corneum containing intercellular lamellar lipidis layers arranged in patterns analogous to those found in vivo. Because it closely mimics the human epidermis, it has proven its scientific relevancy in studies where dermal exposure and toxicity is anticipated (Kandárová H et al., 2009). The parameters measured for skin irritancy assessments of colloidal system of propolis (1%) were the percent MTT cell viability relative to the corresponding negative as well as positive control. According to the MTT assay colloidal system of propolis was proven non skin irritant, showing a cell viability > 50% and confirming the aforementioned results on cell viability. Furthermore, for skin irritancy assessments of colloidal system of propolis based cosmetic formulation we examined the percentage cell viability in the MTT reduction assay with ET₅₀ determination after topical application of the product for different exposure times (2hr,5hr and 18hr). The colloidal system of propolis-based cosmetic formulation was proven mild for skin after 11h of exposure (cell viability>50%).

- For the experimental confirmation of the above, real-time polymerase chain reaction (RT-PCR) (Giulietti A et al., 2001) was employed. For the quantification of the results, two endogenous reference genes were employed, b-actin ACTB and GAPDH. The normalization to the endogenous reference genes is required in order to correct possible differences between samples due to a different concentration of the initial substrate (cDNA) or to differences in the yield of the amplification reaction (Livak KJ et al., 2001).
- From the transcriptomic study it was concluded that the fibroblasts with the colloidal system of 1% propolis presented increased levels on the transcripts of gene endothelial vascular growth factor (VEGFA) in relation to untreated fibroblasts (barchart 2). The vascular growth factor (VEGFA) has been shown to have a major contribution in angiogenesis, increasing the number of capillaries in a network (Moens S et al., 2014).

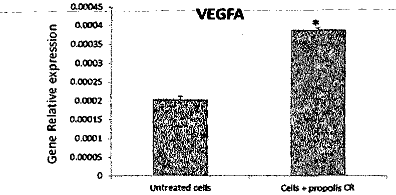

**Barchart 2.** Relative expression levels of the VEGFA gene in fibroplasts with or without a colloidal system of 1% propolis (untreated cells, cells + propolis CR 1%). *Statistically significant difference, Student's t-test (p<0.05).

Also, the colloidal system of 1% propolis has a strong antimicrobial and immunostimulatory action. In order to confirm this, transcripts of genes interleukin-4 (IL4) and integrin B2 (ITGB2) were checked. In particular, an increase in the levels of transcript of gene IL4 in fibroblasts was observed with the colloidal system of 1% propolis in relation to untreated fibroblasts (barchart 3). The increased levels of transcripts of gene IL4 correlates with the activation of the defense pathways against pathogenic microbes on skin fibroblasts (Niebuhr M et al., 2010).

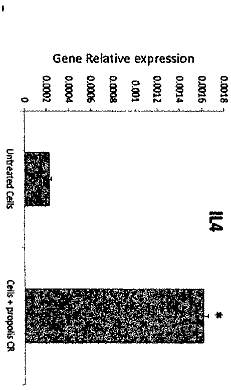

**Barchart 3.** Relative expression levels of the IL-4 gene in fibroplasts with or without a colloidal system of 1% propolis (untreated cells, cells + propolis CR 1%). *Statistically significant difference, Student's t-test (p<0.05).

Finally, by the use of the colloidal system of 1% propolis, an increase on the levels of transcripts of gene ITGB2 in fibroblasts, in relation to untreated fibroblasts, is observed, which demonstrates that its use increases the immune response of the organism, since increased levels of transcripts of gene ITGB2 have been correlated with an immune response (Striz I et al., 1992(barchart 4))

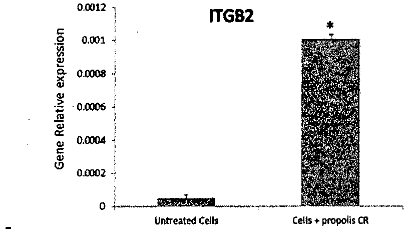

**Barchart 4.** Relative expression levels of the ITGB2 gene in fibroplasts with or without a colloidal system of 1% propolis (untreated cells, cells + propolis CR 1%). *Statistically significant difference, Student's t-test (p<0.05).
Alonso C, Rubio L, Touriño S, Martí M, Barba C, Fernández-Campos F, Coderch L, Parra JL: Antioxidative effects and percutaneous absorption of five polyphenols. Free Radic Biol Med. 2014 Oct; 75:149-55
Abla MJ, Banga AK: Quantification of skin penetration of antioxidants of varying lipophilicity. Int J Cosmet Sci. 2013 Feb;35(1):19-26
Munin A, Edwards-Lévy F: Encapsulation of Natural Polyphenolic Compounds; a Review. Pharmaceutics 2011 Nov 4; 3(4):793-829
De Luca MP, Franca JR, Augusto F, Macedo F, Grenho L, Cortes ME, Faraco GA, Moreira AN, Vagner R. Santos VR: Propolis Varnish: Antimicrobial Properties against Cariogenic Bacteria, Cytotoxicity and Sustained-Release Profile. BioMed Res Int. 2014;2014:348647 Balata G, El Nahas HM, Radwan S: Propolis organogel as a novel topical delivery system for treating wounds: Drug Delivery. 2014 Feb; 21(1):55-61
Franca JR, De Luca MP, Ribeiro TG, Castilho RO, Moreira AN, Santos VR, Faraco AA: Propolis-based chitosan varnish: drug delivery, controlled release and antimicrobial activity against oral pathogen bacteria. MC Complement Altern Med. 2014 Dec; 12(14):478
Zilius M, Ramanauskiene K, Briedis V: Release of propolis phenolic acids from semisolid formulations and their penetration into the human skin in vitro. Evid Based Complement Alternat Med. 2013 ;2013:958717
Butnariu MV, Giuchici CV: The use of some nanoemulsions based on aqueous propolis and lycopene extract in the skin's protective mechanisms against UVA radiation. J Nanobiotechnology. 2011 Feb; 4(9):3
Tao Y, Wang D, Hu Y, Huang Y, Yu Y, Wang D: The immunological enhancement activity of propolis flavonoids liposome in vitro and in vivo. Evid Based Complement Alternat Med. 2014;2014:483513
Yuan J, Lu Y, Abula S, Hu Y, Liu J, Fan Y, Zhao X, Wang D, Liu X, Liu C: Optimization on preparation condition of propolis flavonoids liposome by response surface methodology and research of its immunoenhancement activity. Biomacromolecules. 2013 Mar 11;14(3):854-61
Cutrignelli A, Lopedota A, Denora N, Laquintana V, Tongiani S, Franco M: Characterization and release studies of liposomal gels containing glutathione/cyclodextrins complexes potentially useful for cutaneous administration. J Pharm Sci. 2014 Apr;103(4):1246-54
Kandárová H, Hayden P, Klausner M, Kubilus J, Kearney P, Sheasgreen J. In vitro skin irritation testing: Improving the sensitivity of the EpiDerm skin irritation test protocol. Altern Lab Anim. 2009 Dec;37(6):671-89.
Giulietti A, Overbergh L, Valckx D, Decallonne B, Bouillon R, Mathieu C: An overview of real-time quantitative PCR: applications to quantify cytokine gene expression. Methods. 2001 Dec;25(4):386-401. Review.
Moens S, Goveia J, Stapor PC, Cantelmo AR, Carmeliet P: The multifaceted activity of VEGF in angiogenesis - Implications for therapy responses. Cytokine Growth Factor Rev. 2014 Aug;25(4):473-82.
Niebuhr M, Werfel T. Innate immunity, allergy and atopic dermatitis: Curr Opin Allergy Clin Immunol. 2010 Oct;10(5):463-8.
Striz I, Costabel U: The role of integrins in the immune response. Sarcoidosis. 1992 Sep;9(2):88-94.

## Claims

1. Method for preparing a stable controlled-release propolis colloidal dispersion system, **characterized in that** in order to produce the system, propolis -after being frozen for 24 hours- is micronized (<1 mm) and then dispersed at a rate of 1 kg/min in the solvent system, which is under stirring at 500-3000 rpm and consists of deionized water of <= 1 µS/cm at 25 °C and either natural 1,3-propanediol or glycerol at a mixture ratio 1,3-propanediol (or glycerol)/water from 15%/85% to 80%/20%, in which deionized water hydroxypropyl-β-cyclodextrin or β-cyclodextrin has been predissolved τo 2-10% w/w, and as the propolis/cyclodextrin/solvents system is under intense stirring (2000-3000 rpm) for 30-45 min and at a temperature of 20°C to 38°C, a liposomal suspension is added thereto at a ratio of 0.3 to 3.5% w/w, and stirring is performed for 1 to 4 hours under continuous measurement of the total content in polyphenols by the Folin-Ciocalteu method and of the % encapsulation efficiency, until the specific parameters reach the desired values so that the stirring process is ended and the colloidal system is filtrated through suitable cartridge filters with a pore size of 0.45 µm, its pH is adjusted at the range of 5.0-8.0 and the size of dispersed particles as well as the release rate of the polyphenols of propolis in a buffer solution at pH 7.2 and 37°C are measured and the colloidal system is stored in a dark-coloured container at a temperature of 5-7°C, where it is kept stable for 2 years.

2. Method for preparing a stable controlled-release propolis dispersion colloidal system, according to claim 1, **characterized in that** the propolis used contains total polyphenols >1600 mg/l gallic acid after dissolution of 10 %(w/w) propolis in ethanol and subsequent measurement in a spectrophotometer, its concentration ranging from 7.5% to 28% depending on its polyphenol content.

3. Method for preparing a stable controlled-release propolis dispersion colloidal system, according to claim 1, **characterized in that** the liposomal suspension added consists of large unilamellar liposomes, lipidic bilayers and natural 1,3-propanediol and is at a temperature higher than the phase transition temperature of its phospholipids

4. Method for preparing a stable controlled-release propolis dispersion colloidal system, according to claim 1, **characterized in that** the lipid composition of the liposomal suspension is:
- 50%-90% phosphatidylcholine
- 2%-10% phosphatidylethanolamine
- 1%-3% lysophosphatidylcholine
- 1%-3% phosphatidylinositol
- 1%-3% phosphatidic acid
- 0%-40% cholesterol and
- 0-20% cholate salts.

5. Method for preparing a stable controlled-release propolis dispersion colloidal system, according to claim 1, **characterized in that** the colloidal system is acceptable if its application on NHDF (primary human skin fibroblasts) leads to an increase in their vitality by at least 100% vs. control, which is represented by the increase in ATP (adenosine triphosphate) therein.

6. Method for preparing a stable controlled-release propolis dispersion colloidal system, according to claim 1, **characterized in that** the size of produced liposomes is in the range of 70 to 700 nm and their polydispersity index is less than 0.5 (<0.5), the cumulative release of the polyphenols at pH 7.2 and a temperature of 37°C is 25-60% within 8 hours, while the system releases all encapsulated polyphenols within 24 hours.

## Patentansprüche

1. Verfahren zur Herstellung eines stabilen kolloidalen Propolis-Dispersionssystems mit kontrollierter Freisetzung, das **dadurch gekennzeichnet ist, dass** zur Herstellung des Systems die Propolis nach 24-stündigem Einfrieren mikronisiert (<1 mm) und dann mit einer Geschwindigkeit von 1 kg/min dispergiert wird, im Lösungsmittelsystem, das bei 500-3000 U/min gerührt wird und aus entionisiertem Wasser von <= 1 µS/cm bei 25 °C und entweder natürlichem 1,3-Propandiol oder Glycerin besteht, in einem Mischungsverhältnis von 1,3-Propandiol (oder Glycerin)/Wasser von 15 %/85 % bis 80 %/20 %, in welchem entionisiertem Wasser Hydroxypropyl-β-cyclodextrin oder β-cyclodextrin zu 2-10 % w/w vorgelöst wurde, und während das Propolis/Cyclodextrin/Lösungsmittelsystem 30-45 Minuten lang intensiv gerührt wird, (2000 bis 3000 U/min) bei einer Temperatur von 20 bis 38 °C, wird ihm eine liposomale Suspension in einem Verhältnis von 0,3 bis 3,5 % w/w zugesetzt und es wird für 1 bis 4 Stunden unter kontinuierlicher Messung des Gesamtgehalts an Polyphenolen nach der Folin-Ciocalteu-Methode und der prozentualen Einkapselungseffizienz gerührt, bis die spezifischen Parameter die gewünschten Werte erreicht haben, so dass der Rührvorgang beendet qwird und das kolloidale System durch geeignete Patronenfilter mit einer Porengröße von 0,45 µm filtriert wird. Der pH-Wert wird im Bereich von 5,0 bis 8,0 eingestellt und die Größe der dispergierten Partikel sowie die Freisetzungsrate der Polyphenole von Propolis werden in einer Pufferlösung bei pH 7,2 und 37 °C gemessen und das kolloidale System in einem dunkel gefärbten Behälter bei einer Temperatur von 5 bis 7 °C gelagert, wo es für 2 Jahre lang aufbewahrt wird.

2. Verfahren zur Herstellung eines stabilen kolloidalen Propolis-Dispersionssystems mit kontrollierter Freisetzung nach Anspruch 1, das **dadurch gekennzeichnet ist, dass** die verwendete Propolis nach Auflösung von 10 % (w/w) Propolis in Ethanol und anschließender Messung in einem Spektrophotometer Gesamtpolyphenole> 1600 mg/l Gallussäure enthält; seine Konzentration liegt zwischen 7,5 % und 28 %, abhängig von seinem Polyphenolgehalt.

3. Verfahren zur Herstellung eines stabilen kolloidalen Propolis-Dispersionssystems mit kontrollierter Freisetzung nach Anspruch 1, das **dadurch gekennzeichnet ist, dass** die zugesetzte liposomale Suspension aus großen unilamellaren Liposomen, lipidischen Doppelschichten und natürlichem 1,3-Propandiol besteht und eine Temperatur aufweist, die höher als die Phasenübergangstemperatur seiner Phospholipide ist

4. Verfahren zur Herstellung eines stabilen kolloidalen Propolis-Dispersionssystems mit kontrollierter Freisetzung nach Anspruch 1, das **dadurch gekennzeichnet ist, dass** die Lipidzusammensetzung der liposomalen Suspension wie folgt ist:
- 50 % - 90 % Phosphatidylcholin
- 2 % - 10 % Phosphatidylethanolamin
- 1 % - 3 % Lysophosphatidylcholin
- 1 % - 3 % Phosphatidylinositol
- 1 % - 3 % Phosphatidsäure
- 0 % - 40 % Cholesterin und
- 0 - 20 % Cholatsalze.

5. Verfahren zur Herstellung eines stabilen kolloidalen Propolis-Dispersionssystems mit kontrollierter Freisetzung nach Anspruch 1, das **dadurch gekennzeichnet ist, dass** das kolloidale System akzeptabel ist, wenn seine Anwendung auf NHDF (primäre menschliche Hautfibroblasten) zu einer Steigerung ihrer Vitalität um mindestens 100% führt vs. Kontrolle, die durch die Zunahme von ATP (Adenosintriphosphat) darin dargestellt wird.

6. Verfahren zur Herstellung eines stabilen kolloidalen Propolis-Dispersionssystems mit kontrollierter Freisetzung nach Anspruch 1, das **dadurch gekennzeichnet ist, dass** die Größe der hergestellten Liposomen im Bereich von 70 bis 700 nm liegt und dass ihr Polydispersitätsindex weniger als 0,5 (<0,5) beträgt, dass die kumulative Freisetzung der Polyphenole bei pH 7,2 und einer Temperatur von 37 °C innerhalb von 8 Stunden 25-60 % beträgt, während das System alle eingekapselten Polyphenole innerhalb von 24 Stunden freisetzt.

## Revendications

1. Procédé de préparation d'un système stable de dispersion colloïdale de propolis à libération contrôlée dans lequel le propolis - après avoir été congelé pendant 24 heures - étant micronisé (<1mm) puis dispersé dans le système de solvant au rythme d'1 kg/min, lequel est maintenu sous agitation à 500-3000 tpm et se compose d'eau déionisée de <=1 µs/cm à 25 °C et de 1,3-propanediol naturel ou de glycérol dans un rapport de mélange 1,3-propanediol (ou glycérol)/eau compris entre 15 %/85 % et 80 %/20 %, eau déionisée dans laquelle de l'hydroxypropyl-β-cyclodextrine ou de la β-cyclodextrine a été pré-dissoute à une concentration comprise entre 2 et 10 % en poids, et, alors que le système propolis/cyclodextrine/solvants est soumis à une agitation intense (2000-3000 tpm) pendant 30 à 45 minutes à une température comprise entre 20 °C et 38 °C, une suspension liposomale est ajoutée à ce dernier dans un rapport compris entre 0,3 et 3,5 % en poids, et l'agitation est effectuée pendant 1 à 4 heures sous mesure continue de la teneur totale en polyphénols selon le procédé Folin-Ciocalteu et du pourcentage d'efficacité d'encapsulation, jusqu'à ce que les paramètres spécifiés atteignent les valeurs souhaitées, afin que le processus de mélange puisse être arrêté ; le système colloïdal est ensuite filtré à travers des filtres appropriés avec un pore de 0,45µm, son pH est régulé entre 5.0 et 8,0, et la taille des particules dispersées est mesurée, ainsi que le taux de libération des polyphénols de propolis dans une solution tampon à pH 7,2 et à 37 °C. Le système colloïdal est ensuite conservé dans un récipient de couleur sombre, à une température comprise entre 5 et 7 °C, où il est maintenu stable pendant 2 ans.

2. Procédé de préparation d'un système stable de dispersion colloïdale de propolis à libération contrôlée, conformément à la revendication 1, dans lequel le propolis utilisé contient un total de polyphénols >1600 mg/l d'acide gallique dans lequel de la propolis en éthanol a été pré-dissoute à une concentration de 10 % en poids et mesuré par la suite dans un spectrophotomètre, sa concentration étant entre 7,5% et 28% selon la sa teneur en polyphénol.

3. Procédé de préparation d'un système stable de dispersion colloïdale de propolis à libération contrôlée, conformément à la revendication 1, dans lequel la suspension liposomale ajoutée consiste en de larges liposomes unilamellaires, de bicouches lipidiques et de 1,3-propanediol naturel, et dont la température est supérieure à celle de la phase de transition de ses phospholipides.

4. Procédé de préparation d'un système stable de dispersion colloïdale de propolis à libération contrôlée, conformément à la revendication 1, dans lequel la composition lipidique de la suspension liposomale est la suivante :
- 50%-90% phosphatidylcholine
- 2%-10% phosphatidyléthanolamine
- 1 %-3% lysophosphatidylcholine
- 1 %-3% phosphatidylinositol
- 1 %-3% acide phosphatique
- 0%-40% cholestérol and
- 0-20% sel de cholate.

5. Procédé de préparation d'un système stable de dispersion colloïdale de propolis à libération contrôlée, conformément à la revendication 1, dans lequel le système colloïdal est acceptable si son application sur les fibroblastes primaires de peau humaine (NHDF) a pour effet une augmentation de vitalité à 100% pour le moins vs. contrôle, représentée par une augmentation de l'ATP (triphosphate d'adénosine).

6. Procédé de préparation d'un système stable de dispersion colloïdale de propolis à libération contrôlée, conformément à la revendication 1, dans lequel la taille des liposomes produits est entre 70 à 700 nm et leur Indice de Polydispersité (PDI) est inférieur à 0,5 (<0,5), la libération cumulative des polyphénols sous pH 7,2 et une température de 37°C est entre 25 et 60% dans les 8heures, tandis que le système libère tous les polyphénols encapsulés dans les 24 heures.
